# EUROPÄISCHE PATENTSCHRIFT

(11) **EP 1 017 773 B1**
(45) Veröffentlichungstag und Bekanntmachung des Hinweises auf die Patenterteilung: **05.03.2008**
(21) Anmeldenummer: 98951352.8
(22) Anmeldetag: 05.09.1998
(51) Int. Cl.: C11D 3/30, C11D 3/395, C11D 17/00

(54) **GECOATETE AMMONIUMNITRIL-BLEICHAKTIVATORGRANULATE**
BLEACHING ACTIVATORS BASED ON AMMONIUM NITRILE IN THE FORM OF COATED GRANULES
ACTIVATEURS DE BLANCHIMENT A BASE DE NITRILE D'AMMONIUM SOUS FORME DE GRANULES ENROBES

(30) Priorität: 16.09.1997 DE 19740669
(43) Veröffentlichungstag der Anmeldung: 12.07.2000
(73) Patentinhaber: Clariant Produkte (Deutschland) GmbH, 65929 Frankfurt am Main (DE)
(72) Erfinder: LÖFFLER, Matthias, D-65527 Niedernhausen (DE); REINHARDT, Gerd, D-65779 Kelkheim (DE)
(86) Internationale Anmeldenummer: PCT/EP1998/005629
(87) Internationale Veröffentlichungsnummer: WO 1999/014296

(56) Entgegenhaltungen:
- EP-A- 0 186 052
- EP-A- 0 303 520
- EP-A- 0 458 396
- EP-A- 0 464 880
- EP-A- 0 475 511
- WO-A-92/13798
- WO-A-94/26862
- WO-A-96/40661
- DE-A- 4 232 494
- DE-A- 19 605 526

## Beschreibung

Bleichaktivatoren sind wichtige Bestandteile in Waschmitteln, Fleckensalzen und Maschinengeschirrspülmitteln. Sie ermöglichen eine bleichende Wirkung bereits bei relativ niedrigen Temperaturen, indem sie mit einer Quelle für Wasserstoffperoxid - meist Perborate oder Percarbonate - unter Freisetzung einer organischen Peroxicarbonsäure reagieren bzw. beim Einsatz von Ammoniumnitrilen als Aktivator eine Peroxyimidsäure als bleichendes Agens bilden.

Repräsentative Beispiele für Bleichaktivatoren sind etwa N,N,N',N'-Tetraacetylethylendiamin (TAED), Glucosepentaacetat (GPA), Xylosetetraacetat (TAX), Natrium-4-benzoyloxybenzolsulfonat (SBOBS), Natriumtrimethylhexanoyloxybenzolsulfonat (STHOBS), Tetraacetylglucoluril (TAGU), Tetraacetylcyansäure (TACA), Di-N-acetyldimethylglyoxin (ADMG) und 1-Phenyl-3-acetylhydantoin (PAH). Es sei beispielsweise auf GB-A-836 988, GB-A-907 356, EP-A-0 098 129 und EP-A-0 120 591 verwiesen.

Mittlerweile haben kationische Bleichaktivatoren, die eine quartäre Ammoniumgruppe enthalten, an Bedeutung gewonnen, da sie hocheffektive Bleichaktivatoren sind. Solche kationische Bleichaktivatoren sind beispielsweise in GB-A-1 382 594, US-A-4 751 015, EP-A-0 284 292 und EP-A-0 331 229 beschrieben.

Ammoniumnitrile der Formel 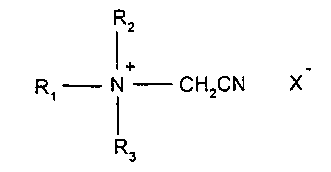 bilden dabei eine besondere Klasse kationischer Bleichaktivatoren. Verbindungen dieser Art und deren Verwendung als Bleichaktivator in Bleichmitteln sind beschrieben in EP-A-303 520, EP-A-464 880, EP-A-458 396 und US-4 883 917. Bei allen dort beschriebenen Verbindungen ist das Stickstoffatom der Ammoniumgruppe durch Alkyl-, Alkenyl- oder Arylgruppen substituiert. Eine weitere Klasse von Ammoniumnitrilen ist in der Deutschen Patentanmeldung 19605526 beschrieben.

Die hohe Reaktivität und Hydrolyseempfindlichkeit der Bleichaktivatoren, insbesondere in Gegenwart alkalischer Waschmittelbestandteile, erfordert jedoch, die genannten Ammoniumnitrile zu granulieren, um eine ausreichende Lagerstabilität zu gewährleisten und um die bleichaktivierende Wirkung erst im Waschgang freizusetzen.

Zur Granulierung dieser Substanzen sind in der Vergangenheit zahlreiche Hilfsstoffe und Verfahren vorgeschlagen worden. In EP-A-0 037 026 wird ein Verfahren zur Herstellung eines leicht löslichen Aktivatorgranulates aus 90 bis 98 % Aktivator mit 10 bis 2 % Celluloseether, Stärke oder Stärkeether beschrieben. Granulate bestehend aus Bleichaktivator, filmbildenden Polymeren und Zusätzen einer organischen C₃-C₆-Carbon-, Hydroxycarbon- oder Ethercarbonsäure werden in WO 90/01535 angeführt. Aus EP-A-0 468 824 sind Granulate aus Bleichaktivator und einem filmbildenden Polymer, das bei pH 10 besser löslich ist als bei pH 7, bekannt. DE-OS-44 39 039 beschreibt ein Verfahren zur Herstellung eines Aktivatorgranulats durch Vermischen eines trockenen Bleichaktivators mit einem trockenen, anorganischen, hydratwasserhaltigen Bindematerial, Verpressen dieser Mischung zu größeren Agglomeraten und Zerkleinern dieser Agglomerate auf die gewünschte Korngröße. Ein wasserfreies Herstellverfahren durch Kompaktierung des Bleichaktivators ohne Einsatz von Wasser mit mindestens einem mit Wasser quellbaren Hilfsstoff ist aus EP-A-0 075 818 bekannt.

Nachteilig bei diesen Aktivatorgranulaten ist, daß die Eigenschaften des Granulates im wesentlichen durch das Bindemittel und das verwendete Granulierverfahren festgelegt sind und oft neben den in der Literatur beschriebenen Vorteilen auch gewisse Nachteile, z.B. nicht optimale Wirkstofffreisetzung, geringe Abriebfestigkeit, hoher Staubanteil, ungenügende Lagerstabilität, Entmischung im Pulver oder Farbschädigung des Gewebes beim Einsatz in Wasch- und Reinigungsmitteln aufweisen.

Um an Granulaten definierte Eigenschaften einzustellen, wird oftmals nachträglich zum Granulierschritt ein Coatingschritt durchgeführt. Gängige Verfahren sind das Coaten in Mischern (mechanisch induzierte Wirbelschicht) oder das Umhüllen in Wirbelschichtapparaturen (pneumatisch induzierte Wirbelschicht).

So wird in WO-92/13798 für einen Bleichaktivator ein Coating mit einer wasserlöslichen oberhalb 30°C schmelzenden organischen Säure und in WO-94/03305 ein Coating mit einem wasserlöslichen sauren Polymer zur Reduzierung der Farbschädigung der Wäsche beschrieben.

Aus WO-94/26862 ist das Coating eines Granulates, bestehend aus Bleichaktivator und wasser- und oder alkalilöslichem Polymer, mit einer zwischen 30°C und 100°C schmelzenden organischen Verbindung zur Reduzierung der Entmischung im pulverförmigen Endprodukt bekannt. Dabei wird das Aktivatorgranulat in einem Lödige-Pflugscharmischer vorgelegt, ohne Verwendung des Zerhackers bei Raumtemperatur mit 160 bis 180 U/min umgewälzt und dann mit der heißen Schmelze besprüht. Nachteil dieses Verfahrens ist eine sehr schlechte Coatingqualität, die zwar eine Reduzierung der Entmischung im pulverförmigen Endprodukt bewirkt, jedoch keinen Einfluß auf die anderen Granulateigenschaften, wie z.B. Wirkstofffreisetzung, Abriebfestigkeit, Staubanteil oder Lagerstabilität hat. Der positive Effekt auf das Entmischungsverhalten ist vermutlich auf ein tröpfchenförmiges Erstarren der Coatingsubstanz auf der Granulatoberfläche zurückzuführen, wodurch ein Verhaken des Granulatkorns im Schüttgut bewirkt wird.

Aufgabe der vorliegenden Erfindung war die Entwicklung eines Coatingverfahrens für Aktivatorgranulate mit einem Gehalt an Ammoniumnitril als Aktivator, das ein gezieltes Einstellen der Granulateigenschaften in einem weiten Bereich bei gleichzeitig optimaler Ausnutzung des Coatingmaterials ermöglicht.

Die Lösung dieser Aufgabe gelang durch ein Coaten mit einer wasserlöslichen Substanz, wobei das so gecoatete Granulat während oder nach dem Coating getempert wird.

Gegenstand der Erfindung sind somit gecoatete Granulate eines Ammoniumnitrils erhältlich durch ein Verfahren, wobei man ein Basisgranulat eines Ammoniumnitrils mit einer wasserlöslichen Coatingsubstanz umhüllt und dieses gecoatete Granulat während des Coatens oder im Anschluß daran tempert.

Für die Basisgranulate können alle Ammoniumnitrile verwendet werden, die in granulierter Form einen Schmelzpunkt oberhalb von 60°C aufweisen. Insbesondere kommen dabei die in der oben erwähnten Literatur beschriebenen Ammoniumnitrile in Frage.

Besonders bevorzugt sind Ammoniumnitrile der Formel 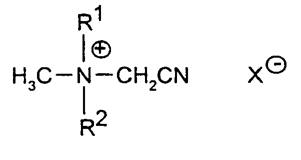 worin R¹ und R² C₁-C₄-Alkyl und X ein Anion, beispielsweise Chlorid oder Methosulfat bedeuten.

Die Basisgranulate können ein oder mehrere dieser Ammoniumnitrile enthalten oder zusätzlich auch Bleichaktivatoren anderen Struktur, beispielsweise N,N,N',N'-Tetraacetylethylendiamin (TAED), Glucosepentaacetat (GPA), Xylosetetraacetat (TAX), Natrium-4-benzoyloxybenzolsulfonat (SBOBS), Natriumtrimethylhexanoyloxybenzolsulfonat (STHOBS), Tetraacetylglucoluril (TAGU), Tetraacetylcyansäure (TACA), Di-N-acetyldimethylglyoxin (ADMG) und 1-Phenyl-3-acetylhydantoin (PAH).

Diese Basisgranulate können die üblichen Granulierhilfsmittel enthalten, welche einen Schmelzpunkt von über 60°C, vorzugsweise über 100°C haben sollen. In Frage kommen hier filmbildende Polymere, z.B.: Celluloseether, Stärke, Stärkeether, Homo-, Co-, Pfropfcopolymerisate ungesättigter Carbonsäuren und/oder Sulfonsäuren sowie deren Salze; Organische Substanzen, z.B.: Cellulose, vernetztes Polyvinylpyrrolidon oder anorganische Substanzen, z.B.: Kieselsäure, amorphe Silikate, Zeolithe, Bentonite, Alkalischichtsilikate der Formel MM'SiₓO₂ₓ₋₁ * y H₂O (M, M' = Na, K, H; x = 1.9 - 23; y = 0 - 25), Ortho-, Pyro-, Polyphosphate, Phosphonsäuren und deren Salze, Sulfate, Carbonate, Bicarbonate. Je nach Bedarf können diese Granulierhilfsmittel als Einzelstoffe oder als Gemische eingesetzt werden.

Neben dem Bleichaktivator und dem Granulierhilfsmittel können die Bleichaktivatorbasisgranulate noch weitere Zusatzstoffe enthalten, die die Eigenschaften wie zum Beispiel Lagerstabilität und Bleichaktivierungsvermögen verbessern. Zu diesen Zusatzstoffen zählen anorganische Säuren, organische Säuren wie etwa ein- oder mehrwertige Carbonsäuren, Hydroxycarbonsäuren und/oder Ethercarbonsäuren sowie deren Salze, Komplexbildner, Metallkomplexe und Ketone. Je nach Bedarf können die vorgenannten Zusatzstoffe als Einzelstoffe oder als Gemische eingesetzt werden.

Die Herstellung dieser Basisgranulate erfolgt durch Vermischen des trockenen Bleichaktivators mit dem trockenen Granulierhilfsmittel, Verpressen dieser Mischung zu größeren Agglomeraten und Zerkleinern dieser Agglomerate auf die gewünschte Korngröße.

Das Gewichtsverhältnis von Bleichaktivator zu Granulierhilfsmittel beträgt üblicherweise 50:50 bis 98:2, vorzugsweise 70:30 bis 96:4. Die Menge der Zusatzstoff richtet sich insbesondere nach deren Art. So werden acidifizierende Zusätze und organische Katalysatoren zur Leistungssteigerung der Persäure in Mengen von 0-20 Gew.-%, insbesondere in Mengen von 1-10 Gew.-%, bezogen auf das Gesamtgewicht, zugesetzt, Metallkomplexe hingegen in Konzentrationen im ppm Bereich.

Als Coatingsubstanzen kommen alle Verbindungen oder deren Mischungen in Frage, die bei Raumtemperatur fest sind und im Bereich von 30 bis 100°C erweichen oder schmelzen. Beispiele hierfür sind:
C₈-C₃₁-Fettsäuren (z.B.: Laurin-, Myristin-, Stearinsäure); C₈-C₃₁-Fettalkohole; Polyalkylenglykole (z.B. Polyethylenglykole mit einer Molmasse von 1000 bis 50000 g/mol); Nonionics (z.B. C₈-C₃₁-Fettalkoholpolyalkoxylate mit 1 bis 100 Molen EO); Anionics (z.B. Alkansulfonate, Alkylbenzolsulfonate, α-Olefinsulfonate, Alkylsulfate, Alkylethersulfate mit C₈-C₃₁-Kohlenwasserstoffresten); Polymere (z.B. Polyvinylalkohole); Wachse (z.B.: Montanwachse, Paraffinwachse, Esterwachse, Polyolefinwachse); Silikone.

In der im Bereich von 30 bis 100°C erweichenden oder schmelzenden Coatingsubstanz können darüber hinaus weitere in diesem Temperaturbereich nicht erweichende oder schmelzende Substanzen in gelöster oder suspendierter Form vorliegen, z.B.: Polymere (z.B. Homo-, Co- oder Pfropfencopolymerisate ungesättigter Carbonsäuren und/oder Sulfonsäuren sowie deren Alkalisalze, Celluloseether, Stärke, Stärkeether, Polyvinylpyrrolidon); Organische Substanzen (z.B. ein- oder mehrwertige Carbonsäuren, Hydroxycarbonsäuren oder Ethercarbonsäuren mit 3 bis 8 C-Atomen sowie deren Salze); Farbstoffe; Anorganische Substanzen (z.B.: Silikate, Carbonate, Bicarbonate, Sulfate, Phosphate, Phosphonate).

Je nach den gewünschten Eigenschaften des gecoateten Aktivatorgranulates kann der Gehalt an Hüllsubstanz 1 bis 30 Gew.-%, bevorzugt 5 bis 15 Gew.-%, bezogen auf gecoatetes Aktivatorgranulat, betragen.

Zum Aufbringen der Hüllsubstanzen können Mischer (mechanisch induzierte Wirbelschicht) und Wirbelschichtapparate (pneumatisch induzierte Wirbelschicht) benutzt werden. Als Mischer sind z.B. Pflugscharmischer (kontinuierlich und chargenweise), Ringschichtmischer oder auch Schugi-Mischer möglich. Die Temperung kann bei Verwendung eines Mischers in einem Granulatvorwärmer und/oder im Mischer direkt und/oder in einem dem Mischer nachgeschalteten Fließbett erfolgen. Zur Kühlung des gecoateten Granulates können Granulatkühler oder Fließbettkühler eingesetzt werden. Im Falle von Wirbelschichtapparaturen erfolgt die Temperung über das zur Aufwirbelung verwendete Heißgas. Das nach dem Wirbelschichtverfahren gecoatete Granulat kann ähnlich wie beim Mischerverfahren über einen Granulatkühler oder einen Fließbettkühler abgekühlt werden. Sowohl beim Mischerverfahren als auch beim Wirbelschichtverfahren kann die Coatingsubstanz über eine Einstoff- oder eine Zweistoffdüsvorrichtung aufgesprüht werden.

Die Temperung besteht in einer Wärmebehandlung bei einer Temperatur von 30 bis 100°C, jedoch gleich oder unterhalb der Schmelz- oder Erweichungstemperatur der jeweiligen Hüllsubstanz. Bevorzugt arbeitet man bei einer Temperatur, die knapp unterhalb der Schmelz- oder Erweichungstemperatur liegt.

Die Korngröße des gecoateten Bleichaktivatorgranulats beträgt von 0.1 bis 2.0 mm, bevorzugt von 0.2 bis 1.0 mm und besonders bevorzugt von 0.3 bis 0.8 mm.

Die genaue Temperatur bei der Temperung bzw. die Temperaturdifferenz zum Schmelzpunkt der Coatingsubstanz ist abhängig von der Coatingmenge, der Temperzeit und den für das gecoatete Bleichaktivatorgranulat gewünschten Eigenschaften und muß für das jeweilige System in Vorversuchen ermittelt werden.

Die Zeitdauer für die Temperung beträgt ungefähr 1 bis 180, vorzugsweise 3 bis 60, besonders bevorzugt 5 bis 30 Minuten.

Der Vorteil dieser Temperung liegt darin, daß das flüssige Coatingmittel nicht zu schnell erstarrt und dadurch die Möglichkeit hat, als dünner Film auf der Granulatoberfläche zu verlaufen. Man erreicht so ein sehr gleichmäßiges Überziehen des Korns in dünner Schicht mit der Coatingsubstanz und einen optimalen Coatingeffekt bei Einsatz einer minimalen Menge Coatingsubstanz. Bei üblichen Verfahren, d.h. ohne Temperungsschritt, tritt ein zu schnelles Erstarren der Einzeltröpfchen auf der kalten Granulatoberfläche ein. Infolgedessen wird die Oberfläche nur mit feinen Einzeltröpfchen belegt und weist noch große Coatingfehlstellen auf. Der gewünschte Coatingeffekt wird dadurch nur unzureichend erzielt bzw. eine deutlich höhere Menge Coatingsubstanz ist zur Erzielung eines gewünschten Coatingeffektes erforderlich. Letzteres reduziert aber den Gehalt an Aktivatorsubstanz, was vielfach unerwünscht ist.

Durch das erfindungsgemäße Verfahren können die Eigenschaften der Ammoniumnitrilgranulate durch geeignete Wahl der Coatingsubstanz, der Coatingmenge und der Temperaturführung des Prozesses in weiten Bereichen gezielt auf die gewünschten Anforderungen hin optimiert werden. Dabei ist vor allem die gezielte Optimierung folgender Aktivatorgranulateigenschaften möglich.
1. Zeitlich optimierte Wirkstofffreisetzung
   Zur Vermeidung der Wechselwirkung zwischen dem Bleichsystem und dem Enzymsystem ist eine zeitlich geringfügig verzögerte Reaktion und Wirkstofffreisetzung des Bleichsystems bei gleichzeitig schneller Enzymwirkung vorteilhaft. Auf diese Weise können die Enzyme in den ersten Minuten des Waschprozesses ihre Waschkraft voll entfalten ohne durch das Bleichsystem geschädigt zu werden. Erst nachdem die Enzyme ihre Arbeit verrichtet haben, wird dann durch Reaktion des Bleichaktivators mit der Wasserstoffperoxidquelle der Bleichprozeß in Gang gesetzt. Durch geeignetes Coating des Bleichaktivators kann die Reaktivität, d.h. die Auflösegeschwindigkeit bzw. die Geschwindigkeit der Persäurebildung gezielt auf das Enzymsystem abgestimmt werden. Bei dem Verfahren ist eine gezielte Einstellung der Persäurebildungsgeschwindigkeit bei gleichzeitig minimaler Menge Coatingsubstanz und daher maximalem Aktivatorgehalt möglich.
2. Erhöhung der Abriebfestigkeit
   Durch Coating eines Granulates mit erweichenden oder schmelzenden Substanzen kann die Abriebfestigkeit eines Aktivatorgranulates erhöht werden. Dabei ist die Erhöhung der Abriebfestigkeit umso größer, je besser die Granulatoberfläche mit der Coatingsubstanz umhüllt ist. Durch das erfindungsgemäße Coating-Verfahren kann mit minimaler Coatingmenge ein optimales Verlaufen der Coating-Substanz auf der Granulatoberfläche und damit eine optimale Verbesserung der Abriebfestigkeit bewirkt werden.
3. Erhöhung der Lagerstabilität
   Bei Lagerung eines Wasch- und Reinigungsmittels kann es an der Grenze zwischen Aktivatorkorn und direkt benachbartem Korn der Wasserstoffperoxidquelle zu einer Reaktion mit anschließendem Aktivsauerstoffverlust und damit zu unkontrolliertem Abbau des Bleichsystems kommen. Durch ein optimales Coating, wie es nur durch das erfindungsgemäße Coatingverfahren möglich ist, wird an der Korngröße eine vollständige Schutzschicht aufgebaut, die dann eine Reaktion des Aktivatorkorns mit dem Korn der Wasserstoffperoxidquelle während der Lagerung unterbindet. Bei Verwendung wasserlöslicher und/oder niedrig schmelzender Coatingsubstanzen kann trotzdem im Waschprozeß die erforderliche Bleichleistung erzielt werden.

Die auf diese Weise erhaltenen Granulate sind direkt zum Einsatz in Wasch- und Reinigungsmitteln geeignet. Sie sind ideal zum Einsatz in Vollwaschmitteln, Fleckensalzen, Maschinengeschirrspülmitteln, pulverförmigen Allzweckreinigern und Gebißreinigern. In diesen Formulierungen werden die erfindungsgemäßen Granulate meist in Kombination mit einer Wasserstoffperoxidquelle eingesetzt. Beispiele dafür sind Perborat-Monohydrat, Perborat-Tetrahydrat, Percarbonate sowie Wasserstoffperoxid-Addukte an Harnstoff oder Aminoxiden. Daneben kann die Formulierung dem Stand der Technik entsprechend weitere Waschmittelbestandteile aufweisen, wie organische oder anorganische Builder und Co-Builder, Tenside, Enzyme, Waschadditive, optische Aufheller und Parfüm.

### Beispiele

### Beispiel 1 Herstellung

In einem 50 l - Lödige-Mischer werden 10 kg einer Mischung aus 92 Gew.-% Ammoniumnitril (Trimethylammoniumacetonitril Toluolsulfonat) und 8 Gew.-% Bentonit (Laundrosil DGA) mit einer Drehzahl von 70 U/min über einen Zeitraum von 10 min intensiv gemischt. Diese homogene Mischung wird anschließend auf einem Walzenkompaktor Pharmapaktor (Fa. Bepex (DE)) mit einer Presskraft von 50 bis 60 kN zu Schülpen verpreßt, die dann in einer zweistufigen Mahlung, Vormahlung mit Zahnscheibenwalzen (Fa. Alexanderwerk (DE)) und Zerkleinerung in einem Passiersieb (Fa. Frewitt (DE)) bei einer Maschenweite von 2000 µm zerkleinert werden.
Man erhält 5.3 kg eines als G1 bezeichneten Granulats mit einer Korngrößenverteilung von 200 bis 1600 µm (Ausbeute: 53 %), sowie 2.8 kg Feinanteil < 200 µm (28 %), die durch erneute Kompaktierung rezykliert werden können, und 1.9 kg Grobanteil > 1600 µm (19 %), die durch erneute Mahlung aufgearbeitet werden können.

### Beispiel 2: Coating nach dem Wirbelschichtverfahren mit nachgeschalteter Temperung

500 bis 600 g Granulat (G1) wurden in der Wirbelschicht (Wirbelschichtapparatur Strea 1, Fa. Aeramatic) vorgelegt und mit einer ca. 80°C heißen Schmelze von Stearinsäure besprüht. Dabei wurde im einen Fall zu Vergleichszwecken das Wirbelbett mit niedrigen Temperaturen betrieben und nach Beendigung des Aufsprühens nochmals für ca. 5 min nachgekühlt. Im anderen Fall wurde nach dem bevorzugten Verfahren das gecoatete Granulat erneut in der Wirbelschicht vorgelegt und getempert. Dazu wurde das Wirbelbett schrittweise auf Temperaturen von etwa 65 bis 70°C erwärmt und diese Produkttemperatur für etwa 5 bis 8 min. konstant gehalten. Anschließend wurde das getemperte Produkt schrittweise wieder abgekühlt.
Die Coatingqualität der Produkte wurde durch Bestimmung der Geschwindigkeit der Persäurebildung bei einer Temperatur von 20°C beurteilt. Je langsamer die Persäurebildung ist, desto besser ist der erzielte Coatinggrad.
Zur Bestimmung der Geschwindigkeit der Persäurebildung wurden in einem 2 l - Becherglas 1 l destilliertes Wasser, 8.0 g Testwaschmittel WMP und 1.5 g Natriumperborat Monohydrat bei 20°C vorgelegt und mit einem Magnetrührer bei 250 bis 280 U/min gerührt. Nach 1 bis 2 min wurden dann 0.5 g des gecoateten Granulates zugegeben. Nach einer Minute wurde ein Aliquot von 50 ml abpipettiert und in einen Erlenmeyerkolben auf 150 g Eis und 5 ml 20 %ige Essigsäure gegeben. Nach Zusatz von 2 bis 3 ml 10 %iger Kaliumiodidlösung wurde zügig mit 0.01 molarer Natriumthiosulfatlösung bis zum potentiometrischen Äquivalenzpunkt titriert (Titroprozessor 716 DMS, Fa. Metrohm) und aus der verbrauchten Menge Natriumthiosulfat die Menge Persäure berechnet. In Abständen von 2 bis 5 min wurden dann weitere Proben entnommen und wie beschrieben titriert. Der ganze Vorgang wurde so oft wiederholt, bis nach drei aufeinanderfolgenden Titrationen gleiche oder sogar fallende Persäuremengen gefunden wurden. Die maximal gefundene Persäuremenge wurde dann zu 100 % gesetzt und auf dieser Basis schließlich die nach 5, 10 und 20 Minuten gebildete Persäuremenge in Prozent als Maß für die Geschwindigkeit der Persäurebildung bestimmt.

**Tabelle 1: Geschwindigkeit der Bildung von Persäure von ungecoateten bzw. im Wirbelschichtverfahren gecoateten Granulaten I mit oder ohne anschließender Temperung**

| gebildete Peressigsäure [%] | 5 min | 10 min | 20 min |
|---|---|---|---|
| G1 (ungecoated) | 70 | 92 | 100 |
| G1 +10 % Stearinsäure, getempert | 11 | 24 | 50 |
| G1 + 20 % Stearinsäure, getempert | 14 | 25 | 54 |
| G1 + 10 % Stearinsäure, ungetempert | 72 | 87 | 97 |
| G1+ 20 % Stearinsäure, ungetempert | 42 | 63 | 82 |

Durch Coaten wird die Freisetzung von Persäure zeitlich stark verzögert. Durch Temperung läßt sich bei gleicher Coatingmenge die Coatingqualität, ausgedrückt durch die Verzögerung der Persäurebildung, deutlich verbessern (Vergleich der Produkte 2 und 4 bzw. Produkte 3 und 5). Zur Erzielung einer optimalen Coatingqualität ist bei geeigneter Temperung eine Menge von 10 % Coatingsubstanz ausreichend (Produkt 2).

### Beispiel 3: Lagerstabilität von Ammoniumnitril-Granulaten in Waschmittelformulierungen

Die Prüfung der Lagerstabilität erfolgte in vorgefertigten Faltschachteln (Höhe-, 6,5 cm; Breite 3,2 cm; Tiefe 2,2 cm) bei 38°C und 80 % relativer Luftfeuchtigkeit (rF) über einen Zeitraum von 28 Tagen. Jede Faltschachtel wurde mit einem homogenen Gemisch aus 8,0 g Testwaschmittel WMP, 1,5 g Natriumpercarbonat und 0,5 g des zu prüfenden Ammoniumnitril-Granulates gefüllt und dann oben mit Tesafilm verschlossen. Alle Proben wurden am gleichen Tag gemischt und abgefüllt. Die gefüllten Faltschachteln wurden dann mit genügend Abstand voneinander in den Klimaschrank gestellt und bei 38°C/80% rF gelagert. Nach 0, 3, 6, 9, 15, 23 und 28 Tagen Lagerzeit wurden die Proben aus dem Klimaschrank entnommen, die Probe bei 20°C in 1 l destilliertes Wasser unter Rühren mit einem Magnetrührer (250 bis 280 U/min) eingetragen und 1 g Natriumpercarbonat zugesetzt. Die weitere Bestimmung der gebildeten Menge an Persäure erfolgte analog den Angaben in Beispiel 2. Aus dem gefundenen Maximalwert der Persäure wurde dann der Ammoniumnitril-Gehalt der Probe berechnet. Der Ammoniumnitril-Erhaltungsgrad stellt den prozentualen Ammoniumnitril-Gehalt der Probe nach Lagerung bezogen auf den Ammoniumnitril-Gehalt der ungelagerten Probe dar.

**Tabelle 2: Lagerstabilität in Waschmittelformulierungen von im Wirbelschichtverfahren gecoateten Ammoniumnitril-Granulaten**

| Ammoniumnitril-Granulat (G1) | | Ammoniumnitril-Erhaltungsgrad nach Lagerung | | | | | |
|---|---|---|---|---|---|---|---|
| | 0d | 3d | 6d | 9d | 15d | 23d | 28d |
| G1 | 100 | 29 | 15 | 12 | 10 | 7 | 6 |
| G1 + 10 % Stearinsäure, getempert | 100 | 88 | 69 | 62 | 57 | 55 | 55 |
| G1 +10% Stearinsäure, ungetempert | 100 | 69 | 35 | 30 | 28 | 26 | 23 |

### Beispiel 4

Die Prüfung der Lagerstabilität der gecoateten Granulate wurde zudem unter praxisnahen Bedingungen in einer Mehrkomponenten-Waschmaschine Öko-Lavamat 6753 (AEG, Nürnberg) an Bleichtestgeweben in Gegenwart reiner Testwäsche geprüft. Entsprechend den Dosiervorschriften für Wasserhärtebereich 3 werden 70 g Referenzwaschmittel (WMP) in die dafür vorgesehenen Einspülkammern der Waschmaschine gegeben. Als Bleichkomponente wurden in die dafür vorgesehene Einspülkammer 8.0 g Percarbonat gegeben sowie
a) 3.18 g Granulat G1 + 10 % Stearinsäure (82 %) gecoatet, getempert
b) 3.18 g Granulat G1 + 10 % Stearinsäure (82 %) gecoatet, nicht getempert.
c) 2.93 g Granulat G1 (92 %) ungecoated

Zum Vergleich wurde Ammoniumnitril Trimethylammoniumacetonitril Toluolsulfonat (2,7 g) in Pulverform geprüft (Bsp. 4d)

Als Ballaststoff werden 2 kg Frotteegewebe verwendet, als Testanschmutzung 10 bleichbare Anschmutzungen (Tee, Rotwein, Curry, Gras der Wäschereiforschung Krefeld). Die Wäsche wurde im Hauptwaschgang bei 40°C gewaschen. Die Auswertung erfolgt durch Bestimmung des Weißgrades nach der Wäsche durch Addition der Remissionsdifferenzen.

**Tabelle 3: Remissionsdifferenzen der Bleichkomponenten a) - d)**

| Bsp.4a) | Bsp.4b) | Bsp.4c) | Bsp.4d) |
|---|---|---|---|
| 352 | 304 | 275 | 50 |

| | | | |
|---|---|---|---|
| T = 40°C, Tee, Rotwein, Curry, Gras | | | |

## Patentansprüche

1. Gecoatete Bleichaktivator-Granulate von Ammoniumnitrilen, erhältlich durch Umhüllen eines Ammoniumnitril-Basisgranulats mit einer wasserlöslichen Coatingsubstanz, **dadurch gekennzeichnet, dass** die Coatingsubstanzen Fettsäuren, Fettalkohole, Polyalkylenglykole, nichtionische Tenside, anionische Tenside, Polymere, Wachse und/oder Silikone sind, die im Bereich von 30 bis 100°C erweichen oder schmelzen und die Granulate während oder nach dem Coatingschritt bei Temperaturen in der Nähe des Erweichungs- oder Schmelzpunkts der Coatingsubstanz getempert werden.

2. Gecoatete Bleichaktivator-Granulate nach Anspruch 1 **dadurch gekennzeichnet, dass** das Granulat ein Ammoniumnitril der Formel 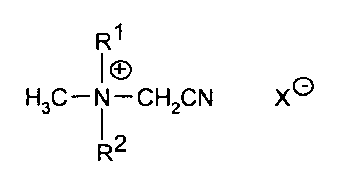 enthält, worin R¹ und R² C₁-C₄-Alkyl und X ein Anion bedeuten.

3. Gecoatete Bleichaktivator-Granulate nach Anspruch 1 oder 2, **dadurch gekennzeichnet, dass** das Ammoniumnitril-Basisgranulat einen Schmelzpunkt von über 100°C aufweist.

4. Gecoatete Bleichaktivator-Granulate nach einem oder mehreren der Ansprüche 1 bis 3, **dadurch gekennzeichnet, dass** die Ammoniumnitril-Basisgranulate zusätzlich einen oder mehrere Bleichaktivatoren enthalten, die keine Ammoniumnitrile darstellen.

5. Gecoatete Bleichaktivator-Granulate nach einem oder mehreren der Ansprüche 1 bis 4, **dadurch gekennzeichnet, dass** die Coatingsubstanz Polymere, organische Substanzen und/oder anorganische Substanzen in gelöster oder suspendierter Form enthält.

6. Gecoatete Bleichaktivator-Granulate nach einem oder mehreren der Ansprüche 1 bis 5, **dadurch gekennzeichnet, dass** der Gehalt an Coatingsubstanz 1 bis 30 Gew.-%, bevorzugt 5 bis 15 Gew.-% beträgt, bezogen auf das gecoatete Bleichaktivatorgranulat.

7. Gecoatete Bleichaktivator-Granulate nach einem oder mehreren der Ansprüche 1 bis 6, **dadurch gekennzeichnet, dass** sie durch Aufbringen der Coatingsubstanz in einem Mischer oder in einem Wirbelschichtapparat erhalten werden.

8. Gecoatete Bleichaktivator-Granulate nach einem oder mehreren der Ansprüche 1 bis 7, **dadurch gekennzeichnet, dass** die Korngröße des gecoateten Bleichaktivatorgranulats 0,1 bis 2,0 mm, bevorzugt von 0,2 bis 1,0 mm und besonders bevorzugt von 0,3 bis 0,8 mm beträgt.

9. Gecoatete Bleichaktivator-Granulate nach einem oder mehreren der Ansprüche 1 bis 8, **dadurch gekennzeichnet, dass** das Basisgranulat bis zu 20 Gew.-% bezogen auf das Gewicht des Basisgranulats ein oder mehrere Zusätze ausgewählt aus der Gruppe bestehend aus anorganischen Säuren, organischen Säuren, Komplexbildnern, Ketonen und Metallkomplexen enthält.

10. Wasch-, Reinigungs-, Bleich- und Desinfektionsmittel, enthaltend ein gecoatetes Bleichaktivatorgranulat, gemäß einem oder mehreren der Ansprüche 1 bis 9.

## Claims

1. Coated bleach activator granules of ammonium nitriles, obtainable by coating ammonium nitrile base granules with a water-soluble coating substance, wherein the coating substances are fatty acids, fatty alcohols, polyalkylene glycols, nonionic surfactants, anionic surfactants, polymers, waxes and/or silicones which soften or melt in the range from 30 to 100°C and the granules are thermally conditioned during or after the coating step at temperatures in the vicinity of the softening or melting point of the coating substance.

2. The coated bleach activator granules as claimed in claim 1, wherein the granules comprise an ammonium nitrile of the formula 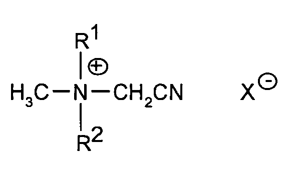 in which R¹ and R² are C₁-C₄-alkyl and X is an anion.

3. The coated bleach activator granules as claimed in claim 1 or 2, wherein the ammonium nitrile base granules have a melting point above 100°C.

4. The coated bleach activator granules as claimed in one or more of claims 1 to 3, wherein the ammonium nitrile base granules additionally comprise one or more bleach activators which are not ammonium nitriles.

5. The coated bleach activator granules as claimed in one or more of claims 1 to 4, wherein the coating substance comprises polymers, organic substances and/or inorganic substances in dissolved or suspended form.

6. The coated bleach activator granules as claimed in one or more of claims 1 to 5, wherein the content of coating substance is from 1 to 30% by weight, preferably from 5 to 15% by weight, based on the coated bleach activator granules.

7. The coated bleach activator granules as claimed in one or more of claims 1 to 6, wherein the coating substance is applied in a mixer or in a fluidized-bed apparatus.

8. The coated bleach activator granules as claimed in one or more of claims 1 to 7, wherein the particle size of the coated bleach activator granules is from 0.1 to 2.0 mm, preferably from 0.2 to 1.0 mm and particularly preferably from 0.3 to 0.8 mm.

9. The coated bleach activator granules as claimed in one or more of claims 1 to 8, wherein the base granules contain up to 20% by weight, based on the weight of the base granules, of one or more additives selected from the group consisting of inorganic acids, organic acids, complexing agents, ketones and metal complexes.

10. A detergent, a cleaning composition, bleach or disinfectant comprising coated bleach activator granules as claimed in one or more of claims 1 to 9.

## Revendications

1. Activateurs de blanchiment à base de nitrile d'ammonium sous forme de granulés enrobés, pouvant être obtenus par enrobage d'un granulé de base de nitrile d'ammonium avec une substance de revêtement hydrosoluble, **caractérisés en ce que** les substances de revêtement sont des acides gras, des alcools gras, des polyalkylèneglycols, des agents tensio-actifs non ioniques, des agents tensio-actifs anioniques, des polymères, des cires et/ou des silicones, qui se ramollissent ou fondent dans la plage de 30 à 100°C et les granulés sont chauffés pendant ou après l'étape de revêtement à des températures au voisinage du point de ramollissement ou de fusion de la substance de revêtement.

2. Activateurs de blanchiment sous forme de granulés enrobés selon la revendication 1, **caractérisés en ce que**, le granulé contient un nitrile d'ammonium de formule 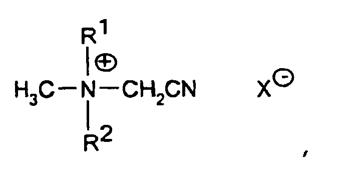 dans laquelle R¹ et R² représentent un alkyle en C₁ à C₄ et X un anion.

3. Activateurs de blanchiment sous forme de granulés enrobés selon la revendication 1 ou 2, **caractérisés en ce que**, le granulé de base de nitrile d'ammonium présente un point de fusion supérieur à 100°C.

4. Activateurs de blanchiment sous forme de granulés enrobés selon une ou plusieurs des revendications 1 à 3, **caractérisés en ce que**, les granulés de base de nitrile d'ammonium contiennent additionnellement un ou plusieurs activateurs de blanchiment, qui ne présentent pas de nitriles d'ammonium.

5. Activateurs de blanchiment sous forme de granulés enrobés selon une ou plusieurs des revendications 1 à 4, **caractérisés en ce que**, la substance de revêtement contient des polymères, des substances organiques et/ou des substances inorganiques sous forme dissoute ou en suspension.

6. Activateurs de blanchiment sous forme de granulés enrobés selon une ou plusieurs des revendications 1 à 5, **caractérisés en ce que**, la teneur en substance de revêtement s'élève à entre 1 et 30 % en poids, de préférence entre 5 et 15 % en poids, par rapport l'activateur de blanchiment sous forme de granulé enrobé.

7. Activateurs de blanchiment sous forme de granulés enrobés selon une ou plusieurs des revendications 1 à 6, **caractérisés en ce que**, ils sont obtenus par dépôt de la substance de revêtement dans un broyeur ou dans un appareil à lit fluidisé.

8. Activateurs de blanchiment sous forme de granulés enrobés selon une ou plusieurs des revendications 1 à 7, **caractérisés en ce que**, la taille des grains de l'activateur de blanchiment sous forme de granulé enrobé s'élève à entre 0,1 et 2,0 mm, de préférence entre 0,2 et 1,0 mm et de manière particulièrement préférée entre 0,3 et 0,8 mm.

9. Activateurs de blanchiment sous forme de granulés enrobés selon une ou plusieurs des revendications 1 à 8, **caractérisés en ce que**, le granulé de base contient jusqu'à 20 % en poids par rapport au poids du granulé de base d'un ou plusieurs additifs choisis dans le groupe constitué des acides inorganiques, des acides organiques, des complexants, des cétones et des complexes métalliques.

10. Lessive, détergent, agent de blanchiment et de désinfection, contenant un activateur de blanchiment sous forme de granulé enrobé, selon une ou plusieurs des revendications 1 à 9.
